# EUROPEAN PATENT SPECIFICATION

(11) **EP 3 031 397 B1**
(45) Date of publication and mention of the grant of the patent: **23.08.2017**
(21) Application number: 15158767.2
(22) Date of filing: 12.03.2015
(51) Int. Cl.: A61B 8/14, A61B 8/08, A61B 8/00

(54) **ULTRASOUND IMAGING APPARATUS**
ULTRASCHALLBILDGEBUNGSVORRICHTUNG
APPAREIL D'IMAGERIE À ULTRASONS

(30) Priority: 28.11.2014 KR 20140168059
(43) Date of publication of application: 15.06.2016
(73) Proprietor: Samsung Medison Co., Ltd., Gangwon-do 250-870 (KR)
(72) Inventor: Han, Gun Hee, Seoul (KR); Kang, Soon Hwan, Gyeonggi-do (KR); Shin, Un Seob, Seoul (KR)
(74) Representative: Frey, Sven Holger

(56) References cited:
- US-A1- 2010 018 314
- US-A1- 2015 025 390

## Description

### BACKGROUND

### 1. Field

Embodiments of the present invention relate to an ultrasound imaging apparatus for generating an image of an interior of a target substance using ultrasound.

### 2. Description of the Related Art

An ultrasound imaging apparatus is an apparatus for obtaining an image of a cross-section of soft tissue or blood flow using information of a reflected ultrasound signal (an ultrasound echo signal) through a non-invasive method of radiating an ultrasound signal from a body surface of a target substance toward a target area in the body.

Ultrasound imaging apparatuses are widely used for cardiac, abdominal, urological and obstetric diagnoses because they are compact and inexpensive, can provide real-time display, and are very safe because there is no exposure to radiation, or the like, in comparison with other image diagnosis apparatuses such as an X-ray diagnosis apparatus, an X-ray computerized tomography (CT) scanner, a magnetic resonance imaging (MRI) scanner, a nuclear medicine diagnosis apparatus, or the like.

An ultrasound imaging apparatus includes an ultrasound probe configured to transmit an ultrasound signal to a target substance and receive an ultrasound echo signal reflected from the target substance to obtain an ultrasound image of the target substance, and a main body configured to generate an image in the target substance using the ultrasound echo signal received by the ultrasound probe.

In the US 2010/018314 A1 an ultrasonic diagnostic equipment is described including: an equipment body; a display unit provided to the equipment body and configured to display an ultrasonic image; an equipment-side connector provided in the equipment body; and an ultrasonic probe having a probe-side connector to be connected to the equipment-side connector in a detachable manner. The ultrasonic probe is connected to the probe-side connector through a cable and configured to send and receive ultrasonic waves. The probe-side connector and the equipment-side connector are formed in structures capable of sending and receiving the ultrasonic waves even in the case of a 180-degree reverse connection of the probe-side connector to the equipment-side connector.

### SUMMARY

Therefore, it is an aspect of the present invention to provide an ultrasound imaging apparatus for generating an ultrasound image normally even when an ultrasound probe is mounted in a reverse direction.

Additional aspects of the invention will be set forth in part in the description which follows and, in part, will be obvious from the description, or may be learned by practice of the invention.

The invention is defined by claim 1.

In accordance with one aspect of the present invention, an ultrasound imaging apparatus according to an embodiment includes an ultrasound probe; a connector configured to connect the ultrasound probe to a main body; and the main body configured to generate an ultrasound image using a signal transmitted through the connector regardless of a direction in which the connector is mounted on the main body.

In addition, the connector includes a sensor configured to recognize a first direction as a normal direction, and output, to the main body, a signal representing that the connector is mounted on the main body in a reverse direction when the connector is mounted on the main body in a second direction opposite to the first direction.

In addition, the sensor includes a gyro sensor.

In addition, the connector may include a plurality of contact sections configured to come in electrical contact with the main body upon mounting on the main body to transmit a signal to the main body, and the contact sections may have a symmetrical array such that the connector is mounted in the slot in different directions symmetrical to each other.

In addition, when the connector is mounted in the second direction, the main body may perform beam forming in a procedure opposite to a beam forming procedure performed with respect to an ultrasound echo signal transmitted through the connector when the connector is mounted in the first direction.

In addition, when the connector is mounted in the second direction, the main body may perform beam forming in a beam forming procedure performed when the connector is mounted in the first direction after the sequence of the ultrasound echo signals transmitted through the connector is switched in a reverse direction.

In addition, when the connector is mounted in the second direction, the main body may perform image processing in a procedure opposite to the image processing procedure performed on the beam forming signal when the connector is mounted in the first direction.

In addition, when the connector is mounted in the second direction, the main body may perform beam forming in a beam forming procedure performed on the ultrasound echo signal transmitted through the connector when the connector is mounted in the first direction.

In addition, the ultrasound probe may perform the beam forming on the ultrasound echo signal to transmit a beam forming signal to the main body through the connector.

### BRIEF DESCRIPTION OF THE DRAWINGS

These and/or other aspects of the invention will become apparent and more readily appreciated from the following description of the embodiments, taken in conjunction with the accompanying drawings of which:
FIG. 1 is a view showing an appearance of an ultrasound imaging apparatus according to an embodiment;
FIG. 2 is a control block diagram of the ultrasound imaging apparatus according to the embodiment;
FIG. 3 is a control block diagram specifically showing a configuration of a main body of the ultrasound imaging apparatus according to the embodiment;
FIG. 4 is a view showing a connector, in which a mechanism is included, and a slot of the ultrasound imaging apparatus according to the embodiment;
FIG. 5 is a view showing a connector, in which an object and a sensor are included, and a slot of the ultrasound imaging apparatus according to the embodiment;
FIGS. 6a and 6b are conceptual views showing that an image processing unit of the ultrasound imaging apparatus according to the embodiment performs image processing differently according to a mounting direction of a connector;
FIG. 6c is a conceptual view showing that the ultrasound imaging apparatus according to the embodiment converts a signal received through the connector in an opposite direction;
FIGS. 7a and 7b are conceptual views showing that a reception beam former of the ultrasound imaging apparatus according to the embodiment performs image processing differently according to a mounting direction of a connector;
FIG. 7c is a conceptual view showing that by the ultrasound imaging apparatus according to the embodiment converts a signal received through the connector into the opposite direction; and
FIGS. 8a and 8b are conceptual views showing that an image processing unit of the ultrasound imaging apparatus according to the embodiment performs image processing differently according to a mounting direction of the connector.

### DETAILED DESCRIPTION

Reference will now be made in detail to the embodiments of the present invention, examples of which are illustrated in the accompanying drawings, wherein like reference numerals refer to like elements throughout.

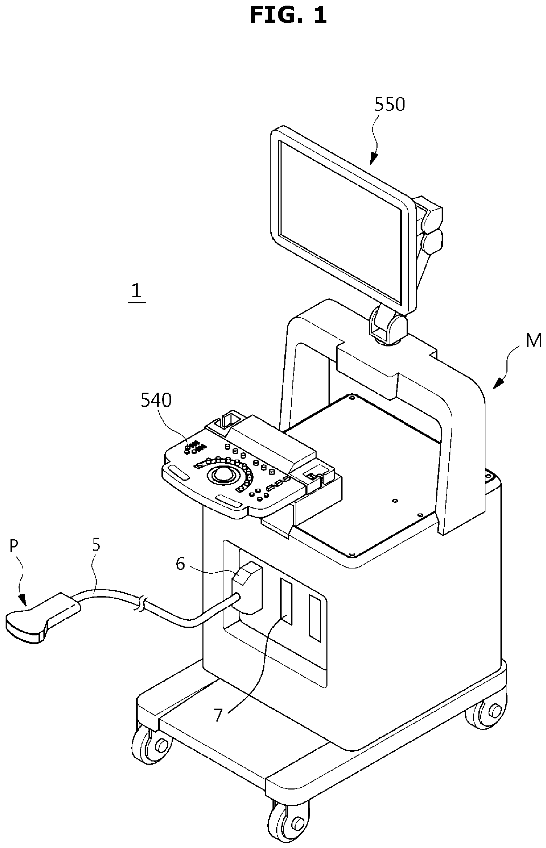

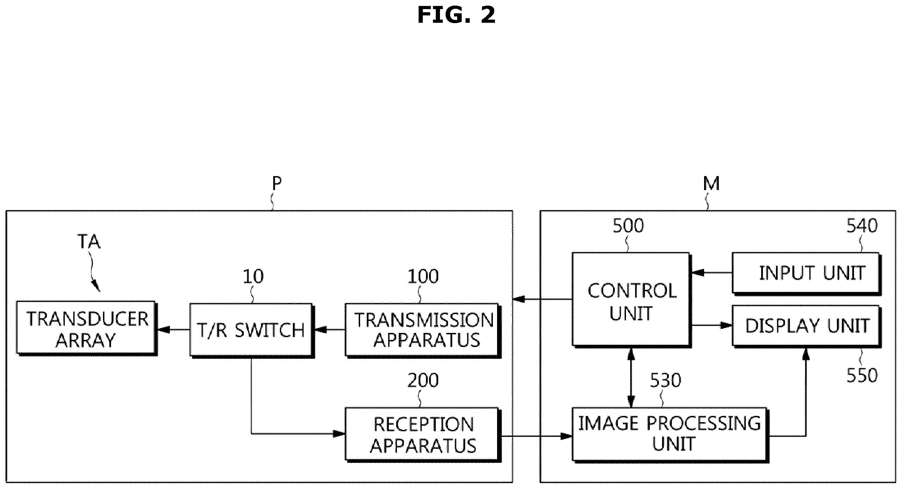

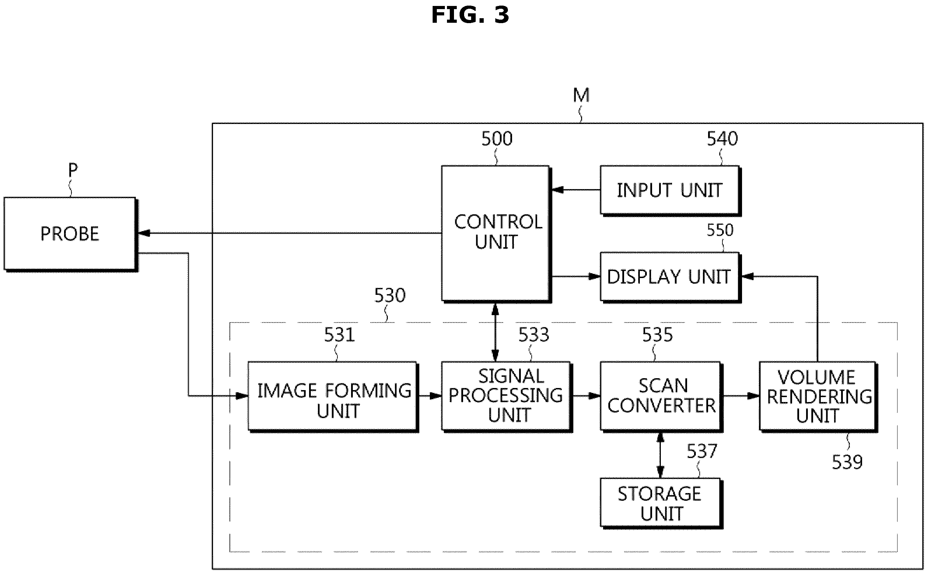

FIG. 1 is a view showing an appearance of an ultrasound imaging apparatus according to an embodiment, and FIG. 2 is a control block diagram of the ultrasound imaging apparatus according to the embodiment. In addition, FIG. 3 is a control block diagram specifically showing a configuration of a main body of the ultrasound imaging apparatus according to the embodiment.

Referring to FIG. 1, an ultrasound imaging apparatus 1 includes an ultrasound probe p configured to transmit ultrasound to a target substance, receive an ultrasound echo signal from the target substance and convert the ultrasound echo signal into an electrical signal, and a main body M connected to the ultrasound probe p, having an input unit 540 and a display unit 550 and configured to display an ultrasound image. An ultrasound probe P can be connected to the main body M of the ultrasound imaging apparatus through a cable 5 to receive various signals required to control the ultrasound probe P, or transmit an analog signal or a digital signal corresponding to the ultrasound echo signal received by the ultrasound probe P to the main body M.

One end of the cable 5 may be connected to the ultrasound probe P, and the other end may be provided with a connector 6 configured to be coupled to or separated from a slot 7 of the main body M. The main body M and the ultrasound probe P can send and receive a control order or data using the cable 5. In general, the connector 6 should be mounted in the slot 7 of the main body in a predetermined direction, i.e., a normal direction. In general, a plurality of pin-shaped contact sections provided to be inserted into the slot 7 are provided at the connector 6, and a plurality of grooves or holes having an array corresponding to an array of pins are provided at the slot 7 such that the pins of the connector 6 can be inserted. Grooves formed in the slot 7 should be connected to the corresponding contact sections of the connector 6 to operate the ultrasound probe P normally, and the main body can generate and display the ultrasound image. That is, the connector 6 should be mounted in the slot 7 of the main body in a predetermined direction, i.e., a normal direction. When the connector 6 is mounted in the slot 7 in a reverse direction opposite to the normal direction, since the grooves of the slot 7 cannot receive the signal from the corresponding contact section, the ultrasound probe P cannot be normally operated, and the main body cannot generate the ultrasound image. In the embodiment, even when the connector 6 is mounted in the reverse direction, the ultrasound imaging apparatus capable of generating the ultrasound image normally regardless of the mounting direction of the connector 6 is provided. Specific description will be provided below.

Meanwhile, as shown in FIG. 2, the main body M may include a control unit 500, an image processing unit 530, the input unit 540 and the display unit 550.

The control unit 500 controls the entire operation of the ultrasound imaging apparatus 1. Specifically, the control unit 500 generates control signals for controlling components of the ultrasound imaging apparatus 1, for example, a transmission apparatus 100, a T/R switch 10, a reception apparatus 200, the image processing unit 530, the display unit 550, and so on, shown in FIG. 2, to control operations of the components. While a transmission/reception beam former is included in the ultrasound probe P rather than in the main body in the ultrasound imaging apparatus according to the embodiment shown in FIGS. 2 and 3, the transmission/reception beam former may be included in the main body rather than in the ultrasound probe P.

The control unit 500 calculates delay profiles of a plurality of ultrasound transducer elements e constituting an ultrasound transducer array TA, and calculates a time delay value according to a distance difference between the plurality of ultrasound transducer elements e included in the ultrasound transducer array TA and a focal point of the target substance based on the calculated delay profiles. In addition, the control unit 500 controls a transmission/reception beam former according to the calculated value to generate a transmission/reception signal.

In addition, the control unit 500 can generate a control order of each of the components of the ultrasound imaging apparatus 1 according to a user's instruction or order input through the input unit 540 to control the ultrasound imaging apparatus 1.

The image processing unit 530 generates an ultrasound image of the target area in the target substance based on the ultrasound signal received through the reception apparatus 200.

Referring to FIG. 3, the image processing unit 530 may include an image forming unit 531, a signal processing unit 533, a scan converter 535, a storage unit 537 and a volume rendering unit 539.

The image forming unit 531 generates a coherent 2-dimensional image or 3-dimensional image of the target area in the target substance based on the ultrasound signal received through the reception apparatus 200.

The signal processing unit 533 converts coherent image information formed by the image forming unit 531 into ultrasound image information according to a diagnosis mode such as a brightness mode (B-mode), a Doppler mode (D-mode), or the like. For example, the signal processing unit 533 performs processing such as A/D conversion processing or the like, and drafts ultrasound image information for a B-mode image in real time when the diagnosis mode is set to the B-mode. In addition, the signal processing unit 533 extracts phase change information from the ultrasound signal, calculates information on blood flow, etc., corresponding to each point of a photographed cross-section such as a speed, power, dispersion, or the like, and drafts ultrasound image information for a D-mode image when the photographing mode is set to the D-mode.

The scan converter 535 converts the converted ultrasound image information received from the signal processing unit 533 or the converted ultrasound image information stored in the storage unit 537 into a general video signal for the display unit 550 to transmit the general video signal to the volume rendering unit 539.

The storage unit 537 temporarily or non-temporarily stores the ultrasound image information converted through the signal processing unit 533.

The volume rendering unit 539 performs volume rendering based on a video signal transmitted from the scan converter 535, compensates the rendered image information to generate a resultant image, and transmits the resultant image to the display unit 550.

The input unit 540 is provided such that a user can input an order to operate the ultrasound imaging apparatus 1. The user can input or set a selection order of diagnosis modes such as an ultrasound diagnosis start order, an A-mode (amplitude mode), a B-mode (brightness mode), a color mode, a D-mode (Doppler mode), an M-mode (motion mode), and so on, region of interest (ROI) setting information including a size and a position of a region of interest (ROI), and so on, through the input unit 540. The input unit 540 may include various means through which the user can input data, an instruction, or an order, for example, a keyboard, a mouse, a trackball, a tablet, a touch screen module, and so on.

The display unit 550 displays a menu or guide required for ultrasound diagnosis, an ultrasound image obtained in an ultrasound diagnosis process, and so on. The display unit 550 displays the ultrasound image of the target area in the target substance generated by the image processing unit 530. The ultrasound image displayed on the display unit 550 may be an ultrasound image of the A-mode, an ultrasound image of the B-mode, or a 3-dimensional ultrasound image. The display unit 550 may be implemented as various known display types such as a cathode ray tube (CRT), a liquid crystal display (LCD), and so on.

As shown in FIG. 2, the ultrasound probe P according to the embodiment may include a transducer array TA, the T/R switch 10, the transmission apparatus 100 and the reception apparatus 200. The transducer array TA is provided at an end of the ultrasound probe p. The ultrasound transducer array TA is an array in which the plurality of ultrasound transducer elements e are arranged in a 1-dimensional or 2-dimensional array. The ultrasound transducer array TA is vibrated by an applied pulse signal or alternating current to generate ultrasound. The generated ultrasound is transmitted to the target area in the target substance. In this case, the ultrasound generated in the ultrasound transducer array TA may be focused on a plurality of target areas in the target substance and transmitted. In other words, the generated ultrasound may be multi-focused on the plurality of target areas and transmitted.

The ultrasound generated in the ultrasound transducer array TA is reflected by the target area in the target substance to return to the ultrasound transducer array TA. The ultrasound transducer array TA receives an ultrasound echo signal reflected and returned from the target area. When the ultrasound echo signal is returned, the ultrasound transducer array TA is vibrated at a predetermined frequency corresponding to a frequency of the ultrasound echo signal, and outputs an alternating current having a frequency corresponding to the vibration frequency. Accordingly, the ultrasound transducer array TA can convert the received ultrasound echo signal into a predetermined electrical signal. Since each of the elements e receives the ultrasound echo signal and outputs the electrical signal, the ultrasound transducer array TA can output electrical signals of a plurality of channels.

The ultrasound transducer may be implemented as one of a magnetostrictive ultrasound transducer using a magnetostrictive effect of a magnetic body, a piezoelectric ultrasound transducer using a piezoelectric effect of a piezoelectric, and a capacitive micromachined ultrasonic transducer (cMUT) for transmitting/receiving ultrasound using vibrations of several hundred or thousand finely-machined thin films. In addition, other kinds of transducers configured to generate ultrasound according to an electrical signal or generate an electrical signal according to ultrasound may also be examples of the ultrasound transducer.

For example, the ultrasound transducer elements e may include a piezoelectric vibrator or a thin film. The piezoelectric vibrator or the thin film is vibrated at a predetermined frequency according to the alternating current when the alternating current is applied from a power source, and generates ultrasound of a predetermined frequency according to the vibration frequency. On the other hand, the piezoelectric vibrator or the thin film is vibrated according to the ultrasound echo signal to output the alternating current of the frequency corresponding to the vibration frequency when the ultrasound echo signal of the predetermined frequency arrives at the piezoelectric vibrator or the thin film.

The transmission apparatus 100 applies a transmission pulse to the transducer array TA to cause the transducer array TA to transmit the ultrasound signal to the target area in the target substance. The transmission apparatus may include a transmission beam former 110 and a pulser 120.

The transmission beam former 110 forms a transmission signal pattern according to a control signal of the control unit 500 of the main body M to output the pattern to the pulser 120. The transmission beam former 110 forms a transmission signal pattern based on a time delay value of each of the ultrasound transducer elements e constituting the ultrasound transducer array TA calculated through the control unit 500 and transmits the formed transmission signal pattern to the pulser 120.

The reception apparatus performs predetermined processing of the ultrasound echo signal received from the transducer array TA, and performs the reception beam forming. The reception apparatus 200 may include a reception signal processing unit and a reception beam former. The electric signal converted in the transducer array TA is input into the reception signal processing unit. The reception signal processing unit can amplify a signal before the ultrasound echo signal performs the signal processing or the time delay processing with respect to the electric signal, and adjust a gain or compensate attenuation according to a depth. More specifically, the reception signal processing unit may include a low noise amplifier (LNA) configured to reduce noise of the electric signal input from the ultrasound transducer array TA, and a variable gain amplifier (VGA) configured to control a gain value according to the input signal. The variable gain amplifier may be a time gain compensator (TGC) configured to compensate the gain according to a distance from the focal point, but is not limited thereto.

The reception beam former performs the beam forming of the electrical signal input from the reception signal processing unit. The reception beam former increases intensity of the signal through superposition of the electrical signals input from the reception signal processing unit. The signal beam-formed in the reception beam former is converted into a digital signal through an analog-digital converter to be transmitted to the image processing unit 530 of the main body M. When the analog-digital converter is provided at the main body M, the analog signal beam-formed in the reception beam former may be transmitted to the main body M to be converted into the digital signal in the main body M. Alternatively, the reception beam former may be a digital beam former. In the case of the digital beam former, the digital beam former may include a storage unit configured to sample and store the analog signal, a sampling period control unit configured to control a sampling period, an amplifier configured to adjust a size of a sample, an anti-aliasing low pass filter configured to prevent aliasing before sampling, a band pass filter configured to select a desired frequency band, an interpolation filter configured to increase sampling upon beam forming, a high-pass filter configured to remove a DC element or a signal of a low frequency band, and so on.

Meanwhile, when the connector 6 is mounted on the main body M in the reverse direction rather than the normal direction, as described above, the ultrasound probe P may not operate normally, or the main body M may be unable to generate the ultrasound image. Accordingly, when the connector 6 is mounted in the reverse direction, the user should mount the connector 6 in the normal direction again. In order to remove inconvenience for the user of having to consider the mounting direction of the connector 6 and mount the connector 6 in the normal direction again when he or she has mounted it in the reverse direction, the embodiment provides the ultrasound imaging apparatus that operates normally even when the user does not consider the mounting direction of the connector 6 and mounts the connector 6 in either of the normal direction and the reverse direction.

The ultrasound imaging apparatus according to the embodiment can recognize that the connector 6 is mounted in the reverse direction rather than the normal direction when the connector 6 is mounted on the main body M in the reverse direction. Here, the normal direction is the mounting direction of the connector 6 in which normal transmission/reception of the data and control order between the ultrasound probe P and the main body M is possible, and the reverse direction is an opposite direction of the normal direction.

The contact sections of the connector 6 have a symmetrical array to be mounted on the main body M in both directions of the normal direction and the reverse direction, and the slots 7 also have a symmetrical array corresponding to the contact sections. For example, provided that the contact sections have a 2-dimensional array of 12×34, when the connector 6 is mounted in the normal direction, a pin located at (1, 1) is mounted in a groove located at (1, 1) corresponding thereto, whereas when the connector 6 is mounted in the reverse direction, a pin located at (12, 34) is mounted in the groove located at (1, 1), and the pin located at (1, 1) is mounted in a groove located at (12, 34).

When the connector 6 is mounted on the main body M in the normal direction, the ultrasound imaging apparatus operates normally, whereas when it is mounted in the reverse direction, a sequence of signals opposite to the sequence of signals received through the connector 6 when it is mounted in the normal direction is transmitted to the main body M. While a normal ultrasound image generation process is performed when the connector 6 is mounted in the normal direction, since an operation of converting the sequence of the ultrasound signals into the opposite direction should be additionally performed when the connector 6 is mounted in the reverse direction, the main body M should detect whether the connector 6 is mounted in the reverse direction.

Hereinafter, various embodiments for detecting whether the connector 6 is mounted in the reverse direction will be described in detail.

The ultrasound imaging apparatus according to the embodiment can use any one of the plurality of contact sections provided in the connector 6 as a discriminator for recognizing the mounting direction of the connector 6. For example, after the connector 6 is mounted on the main body M, when a predetermined signal is transmitted from one contact section (hereinafter referred to as a first contact section) of the plurality of contact sections provided at the connector 6 to the main body M or a predetermined signal is transmitted from the main body M to the first contact section, the main body M can determine that the connector 6 is mounted in the normal direction. On the other hand, after the connector 6 is mounted on the main body M, when the predetermined signal is not transmitted from the first contact section to the main body M or the predetermined signal is not transmitted from the main body M to the first contact section, the main body M can determine that the connector 6 is mounted in the reverse direction.

For example, provided that the contact sections have the 2-dimensional array of 12×34, power of 5 V is supplied to the contact section located at (1, 1), and the contact section located at (12, 34) may be designed as ground. The main body M determines that the connector 6 is mounted in the normal direction when a power signal of 5 V is applied to the contact section located at (1, 1) after the connector 6 is mounted, and determines that the connector 6 is mounted in the reverse direction when the power signal of 5 V is not applied to the contact section located at (1, 1).

The above-mentioned description is only exemplary and at least one contact section may be used to determine the mounting direction of the connector 6. As described above, a separate contact section used only to determine the mounting direction of the connector 6 separate from the contact section configured to perform an inherent function like the contact section related to supply of the power may be provided to determine the mounting direction of the connector 6.

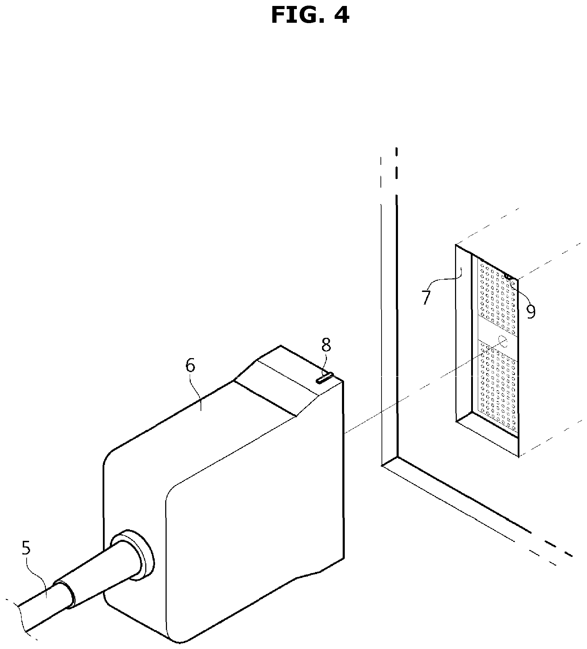

In addition, the slot 7 of the main body M according to the embodiment may include a mechanism 9 configured to generate a structural change when the connector 6 is mounted in the normal direction. FIG. 4 is a view showing the connector 6, in which the mechanism is included, and the slot 7 of the ultrasound imaging apparatus according to the embodiment. For example, as shown in FIG. 4, the mechanism 9 having a structure such as a switch or a button pushed when a pressure is applied may be installed in the slot 7. The mechanism 9 can output a predetermined signal when pushed by the pressure, and the main body M can determine that the connector 6 is mounted in the normal direction when the signal output from the mechanism 9 is received and determine that the connector 6 is mounted in the reverse direction when the signal is not received. Another mechanism 8 may be provided at the connector 6 to come in contact with the mechanism 9 to apply a predetermined pressure to the mechanism 9 upon mounting of the connector 6 in the normal direction such that the pressure is applied to the mechanism 9 of the slot 7 only when the connector 6 is mounted in the normal direction. The mechanism 8 provided at the connector 6 may be provided at a position at which the pressure is applied to the mechanism 9 of the slot 7 when the connector 6 is mounted in the normal direction. While the mechanism 9 of the slot 7 is structurally changed to be pushed by the pressure or the like, since the mechanism 8 of the connector 6 applies the pressure, a structure fixed not to move upon coming in contact with the mechanism 9 of the slot 7 may be provided.

An embodiment operated in a reverse manner is also possible. That is, when the connector 6 is mounted in the normal direction, the mechanism 8 installed at the connector 6 may be provided to be pushed, and the mechanism 9 provided in the slot 7 may be provided to apply a pressure to the mechanism 8 of the connector 6.

In addition, the mechanism of the connector 6 or the slot 7 may be implemented to generate a structural change when the connector 6 is mounted in the reverse direction rather than the normal direction.

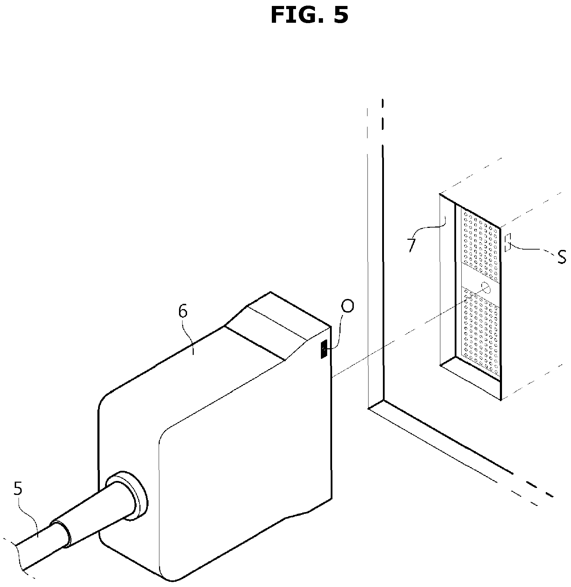

In addition, the slot 7 of the main body M according to the embodiment may include a sensor S configured to detect when the connector 6 is mounted in the normal direction. FIG. 5 is a view showing the connector 6, in which an object O and the sensor S are included, and the slot 7 of the ultrasound imaging apparatus according to the embodiment. The connector 6 may include the object O provided to be detected by the sensor S of the slot 7 when the connector 6 is mounted in the normal direction. That is, when the connector 6 is mounted in the normal direction, the sensor S can detect the object O of the connector 6 to output a predetermined signal, and the main body M can receive the signal output from the sensor S to determine that the connector 6 is mounted in the normal direction. In addition, when the signal is not output from the sensor S, the main body M can determine that the connector 6 is mounted in the reverse direction.

On the other hand, when the sensor S is installed at the connector 6 and the connector 6 is mounted in the normal direction, the object O that can be detected by the sensor S of the connector 6 may be installed at the slot 7. The sensor S of the connector 6 can output a detection signal to the main body M when the object O of the slot 7 is detected, and the main body M can receive the detection signal to determine that the connector 6 is mounted in the normal direction.

As described above, when the connector 6 is mounted in the reverse direction, the sensor S and the object O may be installed at the connector 6 and the slot 7 such that the sensor S detects the object O to output the signal. In this case, the main body M determines the mounting direction of the connector 6 as the reverse direction when the signal is output from the sensor S, and determines the mounting direction of the connector 6 as the normal direction when the signal is not output.

In addition, the mounting direction of the connector 6 can be detected by installing only the sensor S without the object O. For example, when a gyro sensor is installed at the connector 6, if a normal mounting direction of the connector 6 is set as a reference direction and the connector 6 is mounted in the reverse direction, the gyro sensor can output the signal. When the signal is output from the gyro sensor, the main body M receives the signal to determine the mounting direction of the connector 6 as the reverse direction. On the other hand, when a reverse mounting direction of the connector 6 is set as a reference direction and the connector 6 is mounted in the normal direction, the signal may be output from the gyro sensor. In this case, when the signal is not output from the gyro sensor, the main body M determines the mounting direction of the connector 6 as the reverse direction. The gyro sensor is merely an example, and various kinds of sensors configured to detect the mounting direction of the connector 6 without the separate object O may be used.

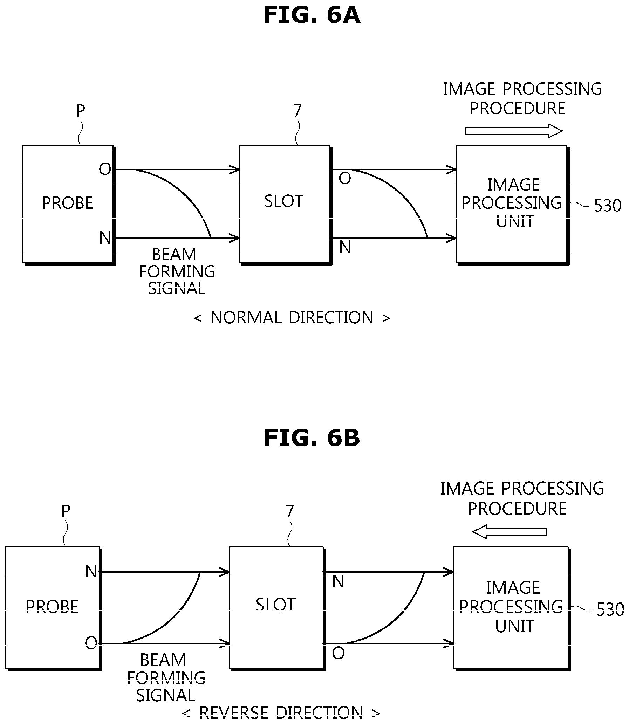

When the connector 6 is mounted in the reverse direction, a sequence of the signals transmitted through the connector 6 is opposite to a sequence of the signals transmitted through the connector 6 when the connector 6 is mounted in the normal direction. For example, when the connector 6 is mounted in the normal direction, the groove located at (5, 1) of the slot 7 receives the signal transmitted from the contact section located at (5, 1) of the connector 6. However, when the connector 6 is mounted in the reverse direction, the groove located at (5, 1) of the slot 7 receives the signal transmitted from the contact section located at (8, 34) of the connector 6, and the groove located at (8, 34) of the slot 7 receives the signal transmitted from the contact section located at (5, 1) of the connector 6. Accordingly, when the mounting direction of the connector 6 is determined as the reverse direction, the main body M should convert the signal received through the connector 6 into the sequence of signals received when the connector 6 is mounted in the normal direction. Hereinafter, referring to FIGS. 6a to 6c, a method of generating an ultrasound image in the main body M when the connector 6 of the ultrasound probe P including the transmission/reception beam former is installed in the reverse direction will be described in detail.

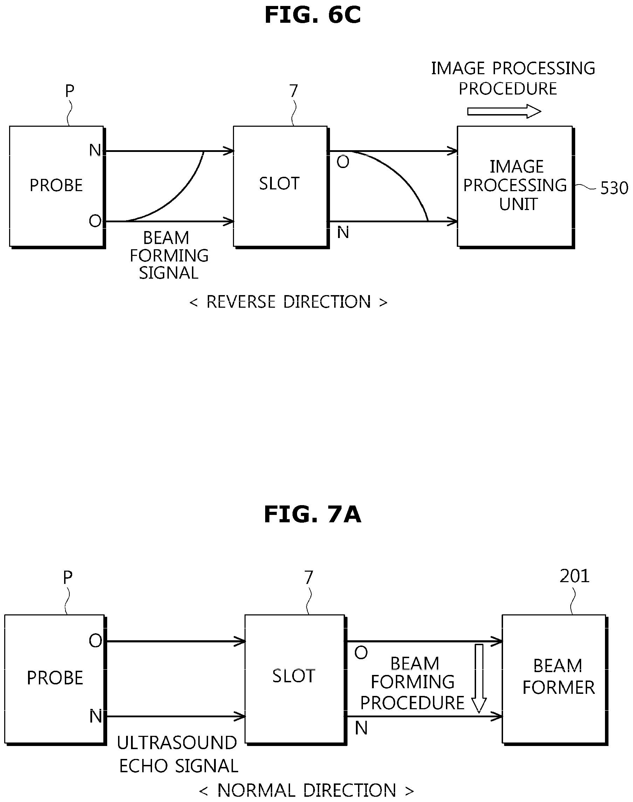

FIGS. 6a and 6b are conceptual views showing that the image processing unit 530 of the ultrasound imaging apparatus according to the embodiment performs the image processing differently according to the mounting direction of the connector 6, and FIG. 6c is a conceptual view showing that the ultrasound imaging apparatus according to the embodiment converts the signal received from the connector 6 into the opposite direction.

When the ultrasound probe P includes the transmission/reception beam former, the signal transmitted through the connector 6 is a beam forming signal obtained by performing the reception beam forming. FIGS. 6a to 6c show curves symbolically representing time delay applied to the ultrasound echo signal to discriminate the beam forming signal from the ultrasound echo signal before beam forming.

FIG. 6a shows an image processing procedure performed on the beam forming signal transmitted through the connector 6 in which the image processing unit 530 is mounted in the normal direction when the connector 6 is mounted in the normal direction. On the other hand, FIG. 6b shows an image processing procedure performed on the beam forming signal by the image processing unit 530 when the connector 6 is mounted in the reverse direction. As shown in FIG. 6b, the image processing unit 530 of the main body M performs the image processing in an opposite procedure of the image processing procedure shown in FIG. 6a with respect to the sequence of the beam forming signal to generate the ultrasound image when the beam forming signal transmitted through the connector 6 mounted in the reverse direction is received. The image processing may be performed in the opposite procedure with respect to the beam forming signal in the image processing unit 530, and as shown in FIG. 6c, after the sequence of the beam forming signals before the beam forming signals are input into the image processing unit 530 is converted into the sequence of the beam forming signals transmitted when the connector 6 is mounted in the normal direction, the beam forming signal of the converted sequence can be transmitted to the image processing unit 530. For example, a relay device of a board provided in the slot 7 can be controlled by the control unit of the main body M to convert the sequence of the beam forming signals into the sequence of the beam forming signals when the connector 6 is mounted in the normal direction. In this case, the image processing unit 530 performs the image processing like the case in which the connector 6 is mounted in the normal direction to thereby generate the ultrasound image.

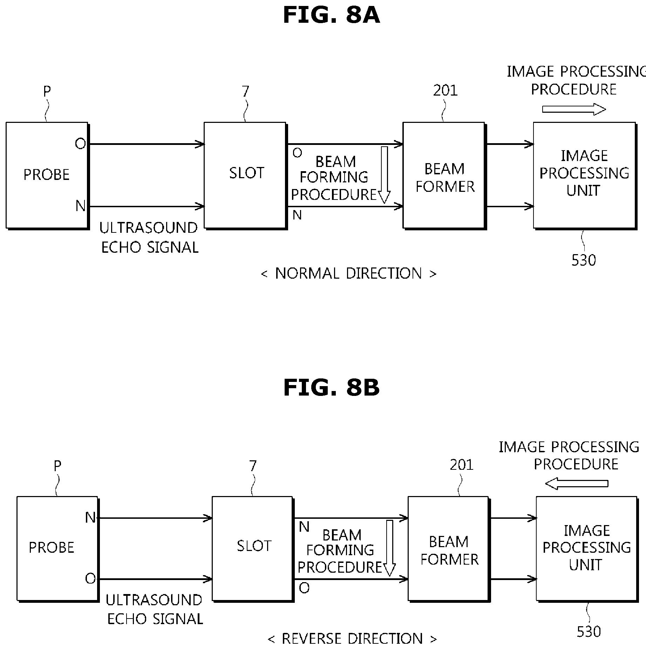

When the transmission/reception beam former is provided in the main body M rather than in the ultrasound probe P, the ultrasound echo signal received by the element of the ultrasound transducer array TA is transmitted to the main body M through the connector 6. The main body M performs the predetermined signal processing and reception beam forming with respect to the ultrasound echo signal transmitted through the connector 6 to generate the ultrasound image. Hereinafter, a method of generating the ultrasound image in the main body M including the transmission/reception beam former when the connector 6 is mounted in the reverse direction will be described in detail with reference to FIGS. 7a to 7c, 8a and 8b.

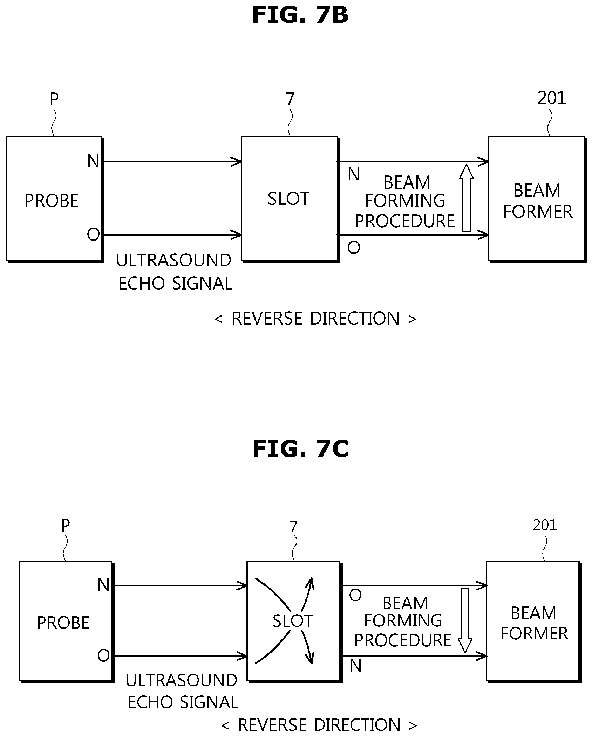

FIGS. 7a and 7b are conceptual views showing that a reception beam former 201 of the ultrasound imaging apparatus according to the embodiment performs the image processing differently according to the mounting direction of the connector 6, and FIG. 7c is a conceptual view showing that the ultrasound imaging apparatus according to the embodiment converts the signal received through the connector 6 into the opposite direction. FIGS. 8a and 8b are conceptual views showing that the image processing unit 530 of the ultrasound imaging apparatus according to the embodiment performs the image processing differently according to the mounting direction of the connector 6.

FIG. 7a shows the beam forming procedure performed on the ultrasound echo signal transmitted through the connector 6 in which the reception beam former 201 is mounted in the normal direction, when the connector 6 is mounted in the normal direction. On the other hand, FIG. 7b shows the beam forming procedure performed on the ultrasound echo signal by the reception beam former 201 when the connector 6 is mounted in the reverse direction. As shown in FIG. 7b, when the connector 6 is mounted in the reverse direction, the main body M performs the reception beam forming in an opposite procedure of the beam forming procedure shown in FIG. 7a with respect to the sequence of the ultrasound echo signal transmitted through the connector 6 mounted in the reverse direction to generate the beam forming signals of the same sequence as the beam forming signals output from the reception beam former 201 when the connector 6 is mounted in the normal direction. In this case, the image processing unit 530 performs the image processing like the case in which the connector 6 is mounted in the normal direction to thereby generate the ultrasound image.

As another example, as shown in FIG. 7c, after the sequence of the ultrasound echo signals before the ultrasound echo signal is input into the reception beam former 201 of the main body M is converted into the sequence of the ultrasound echo signal transmitted when the connector 6 is mounted in the normal direction, the ultrasound echo signals of the converted sequence can be transmitted to the reception beam former 201. For example, the relay device of the board provided in the slot 7 can be controlled in the control unit of the main body M to convert the sequence of the ultrasound echo signals into the sequence of the ultrasound echo signals when the connector 6 is mounted in the normal direction. In this case, the reception beam former 201 performs the beam forming like the case in which the connector 6 is mounted in the normal direction to thereby generate the ultrasound image.

FIG. 8a shows a beam forming procedure performed on the ultrasound echo signal transmitted through the connector 6 in which the reception beam former 201 is mounted in the normal direction when the connector 6 is mounted in the normal direction, and an image processing procedure performed on the beam forming signal generated in the reception beam former 201 by the image processing unit 530. On the other hand, FIG. 8b shows an image processing procedure performed on the beam forming signal by the image processing unit 530 when the connector 6 is mounted in the reverse direction. As shown in FIG. 8b, even when the connector 6 is mounted in the reverse direction, the reception beam former 201 performs the reception beam forming like when mounted in the normal direction without converting the sequence of the ultrasound echo signals transmitted through the connector 6 mounted in the reverse direction, and the image processing may be performed in the image processing unit 530 in an opposite procedure of the sequence of the beam forming signal to generate the ultrasound image.

That is, when the connector 6 is mounted in the reverse direction, as shown in FIG. 7b, the reception beam forming may be performed in an opposite procedure of the sequence of the ultrasound echo signals in the reception beam former 201, or as shown in FIG. 8b, the image processing may be performed in the image processing unit 530 in an opposite procedure of the sequence of the beam forming signals to generate the normal ultrasound image.

As is apparent from the above description, the ultrasound imaging apparatus according to the aspect of the present invention can generate the normal ultrasound image regardless of a direction in which the ultrasound probe is mounted on the main body.

In addition, since the ultrasound imaging apparatus operates normally regardless of the mounting direction of the ultrasound probe, the user has no need to check the mounting direction of the ultrasound probe.

Although a few embodiments of the present invention have been shown and described, it would be appreciated by those skilled in the art that changes may be made in these embodiments without departing from the principles and spirit of the invention, the scope of which is defined in the claims and their equivalents.

## Claims

1. An ultrasound imaging apparatus (1) comprising:
an ultrasound probe (P);
a connector (6) configured to connect the ultrasound probe (P) to a slot (7) of a main body (M); and
the main body (M) configured to generate an ultrasound image using a signal transmitted through the connector (6) mounted in the slot (7) in a first and a second direction, wherein the first direction is a predetermined normal direction and the second direction is a reverse direction opposite to the first direction, **characterized in that** the connector (6) comprises a sensor configured to recognize the first direction, and when the connector (6) is mounted on the main body (M) in the second direction, output, to the main body (M), a signal representing that the connector (6) is mounted on the main body (M) in the second direction, and wherein the sensor comprises a gyro sensor.

2. The ultrasound imaging apparatus (1) according to claim 1, wherein the connector (6) comprises a plurality of contact sections configured to come in electrical contact with the corresponding contact sections of the slot (7) of the main body (M) upon mounting on the main body (M) to transmit a signal to the main body (M), and the contact sections have a symmetrical array such that the connector (6) can be mounted in the slot (7) in the first and second direction.

3. The ultrasound imaging apparatus (1) according to claim 2, wherein the main body (M) is configured to perform beam forming, when the connector (6) is mounted in the second direction, opposite to a beam forming performed with respect to an ultrasound echo signal transmitted through a connector (6) mounted in the first direction.

4. The ultrasound imaging apparatus (1) according to claim 2, wherein the main body (M) is configured to perform beam forming, when the connector (6) is mounted in the second direction, as in the first direction and then to switch the sequence of the ultrasound echo signals transmitted through the connector (6) in a reverse direction.

5. The ultrasound imaging apparatus (1) according to claim 2, wherein, when the connector (6) is mounted in the second direction, the main body (M) is configured to perform image processing in a procedure opposite to the image processing procedure performed on the beam forming signal when a connector (6) is mounted in the first direction.

6. The ultrasound imaging apparatus (1) according to claim 5, wherein, when the connector (6) is mounted in the second direction, the main body (M) is configured to perform beam forming on the ultrasound echo signal transmitted through the connector (6).

7. The ultrasound imaging apparatus (1) according to claim 5, wherein the ultrasound probe is configured to perform the beam forming on the ultrasound echo signal transmitted to the main body (M) through the connector (6).

## Patentansprüche

1. Ultraschall-Abbildungsvorrichtung (1), welche Folgendes aufweist:
eine Ultraschallsonde (P);
einen Verbinder (6), der dafür vorgesehen ist, die Ultraschallsonde (P) mit einem Schlitz (7) eines Hauptkörpers (M) zu verbinden; und
wobei der Hauptkörper (M) dafür vorgesehen ist, ein Ultraschallbild unter Verwendung eines Signals zu erzeugen, das durch den in dem Schlitz (7) montierten Verbinder (6) in einer ersten und in einer zweiten Richtung übertragen wurde, wobei die erste Richtung eine vorbestimmte Normalrichtung und die zweite Richtung eine Rückwärtsrichtung gegenüberliegend zu der ersten Richtung ist,
**dadurch gekennzeichnet, dass**
der Verbinder (6) einen Sensor aufweist, der dafür vorgesehen ist, die erste Richtung zu erkennen, und wenn der Verbinder (6) in der zweiten Richtung an dem Hauptkörper (M) montiert ist, an den Hauptkörper (M) ein Signal auszugeben, dass der Verbinder (6) in der zweiten Richtung an dem Hauptkörper (M) montiert ist, und wobei der Sensor einen Gyrosensor aufweist.

2. Ultraschall-Abbildungsvorrichtung (1) nach Anspruch 1, wobei der Verbinder (6) eine Vielzahl von Kontaktabschnitten aufweist, die dafür vorgesehen sind nach dem Montieren an dem Hauptkörper (M), in elektrischen Kontakt mit den entsprechenden Kontaktabschnitten des Schlitzes (7) des Hauptkörpers (M) zu kommen, um ein Signal an den Hauptkörper (M) zu übertragen, und wobei die Kontaktabschnitte eine derartige symmetrische Anordnung aufweisen, dass der Verbinder (6) in der ersten und in der zweiten Richtung in dem Schlitz (7) montiert werden kann.

3. Ultraschall-Abbildungsvorrichtung (1) nach Anspruch 2, wobei der Hauptkörper (M) dafür vorgesehen ist, eine Strahlumformung durchzuführen, wenn der Verbinder (6) in der zweiten Richtung montiert ist, und zwar entgegengesetzt zu einer Strahlumformung, die unter Bezugnahme auf ein Ultraschallechosignal durchgeführt wird, das durch den in der ersten Richtung montierten Verbinder (6) übertragen wird.

4. Ultraschall-Abbildungsvorrichtung (1) nach Anspruch 2, wobei der Hauptkörper (M) dafür vorgesehen ist, eine Strahlumformung durchzuführen, wenn der Verbinder (6) in der zweiten Richtung montiert ist, und zwar wie in der ersten Richtung, und dann die Sequenz der Ultraschallechosignale, die durch den Verbinder (6) übertragen wurden, in eine Rückwärtsrichtung umzuschalten.

5. Ultraschall-Abbildungsvorrichtung (1) nach Anspruch 2, wobei, wenn der Verbinder (6) in der zweiten Richtung montiert ist, der Hauptkörper (M) dafür vorgesehen ist, eine Bildverarbeitung in einer Vorgehensweise durchzuführen, die entgegengesetzt zu der Vorgehensweise bei der Bildverarbeitung ist, die an dem Strahlumformsignal durchgeführt wird, wenn der Verbinder (6) in der ersten Richtung montiert ist.

6. Ultraschall-Abbildungsvorrichtung (1) nach Anspruch 5, wobei, wenn der Verbinder (6) in der zweiten Richtung montiert ist, der Hauptkörper (M) dafür vorgesehen ist, eine Strahlumformung an dem Ultraschallechosignal durchzuführen, das durch den Verbinder (6) übertragen wurde.

7. Ultraschall-Abbildungsvorrichtung (1) nach Anspruch 5, wobei die Ultraschallsonde dafür vorgesehen ist, die Strahlumformung an dem Ultraschallechosignal durchzuführen, das durch den Verbinder (6) zu dem Hauptkörper (M) übertragen wurde.

## Revendications

1. Appareil d'imagerie ultrasonique (1) comportant :
une sonde ultrasonique (P);
un connecteur (6) configuré pour connecter la sonde ultrasonique (P) à une fente (7) d'un corps principal (M) ; et ;
un corps principal (M) configuré pour générer une image ultrasonique utilisant un signal transmis par le connecteur (6) monté dans la
fente (7) dans une première et dans une seconde directions, dans lequel la première direction est une direction normale prédéterminée et la seconde direction est une direction inverse opposée à la première direction,
**caractérisé en ce que** le connecteur (6) comporte un capteur configuré pour reconnaître la première direction, et lorsque le connecteur (6) est monté sur le corps principal (M) dans la seconde direction, pour émettre vers le corps principal (M), un signal qui montre que le connecteur (6) est monté sur le corps principal (M) dans la seconde direction, et
dans lequel le capteur comporte un capteur gyroscopique.

2. Appareil d'imagerie ultrasonique (1) selon la revendication 1, dans lequel le connecteur (6) comporte une pluralité de sections de contact configurées pour entrer en contact électrique avec les sections de contact correspondantes de la fente (7) du corps principal (M) suite au montage sur le corps principal (M), pour transmettre le signal au corps principal (M), et les sections de contact ayant une disposition symétrique telle que le connecteur (6) peut être monté dans la fente (7) dans la première et dans la seconde directions.

3. Appareil d'imagerie ultrasonique (1) selon la revendication 2, dans lequel le corps principal (M) est configuré pour réaliser la formation d'un rayon, lorsque le connecteur (6) est monté dans la seconde direction, opposée à une formation de rayon réalisée selon un signal d'écho ultrasonique par un connecteur (6) monté dans la première direction.

4. Appareil d'imagerie ultrasonique (1) selon la revendication 2, dans lequel le corps principal (M) est configuré pour réaliser la formation d'un rayon, lorsque le connecteur (6) est monté dans la seconde direction, ainsi que dans la première direction, et ensuite pour commuter la séquence des signaux d'écho ultrasoniques transmis à travers le connecteur (6) dans une direction inverse.

5. Appareil d'imagerie ultrasonique (1) selon la revendication 2, dans lequel, lorsque le connecteur (6) est monté dans la seconde direction, le corps principal (M) est configuré pour réaliser un traitement d'images au cours d'une procédure opposée par rapport à un processus de traitement d'image effectuée sur le signal de formation de rayon, lorsqu'un connecteur (6) est monté dans la première direction.

6. Appareil d'imagerie ultrasonique (1) selon la revendication 5, dans lequel, lorsque le connecteur (6) est monté dans la seconde direction, le corps principal (M) est configuré pour réaliser la formation d'un rayon sur le signal d'écho ultrasonique transmis par le connecteur (6).

7. Appareil d'imagerie ultrasonique (1) selon la revendication 5, dans lequel, la sonde ultrasonique est configurée pour former un rayon sur le signal d'écho ultrasonique transmis au corps principal (M) à travers le connecteur (6).
